Europäisches Patentamt

European Patent Office

Office européen des brevets

(19)

(11) Veröffentlichungsnummer: **0 411 306 A1**

# (12) EUROPÄISCHE PATENTANMELDUNG

(21) Anmeldenummer: **90111920.6**

(22) Anmeldetag: **22.06.90**

(51) Int. Cl.5: **G01N 33/68**, G01N 33/577, C12N 5/12, C12P 21/08, A61K 39/395

(30) Priorität: **27.07.89 DE 3924924**

(43) Veröffentlichungstag der Anmeldung: **06.02.91 Patentblatt 91/06**

(84) Benannte Vertragsstaaten: **BE CH DE FR GB IT LI NL SE**

(71) Anmelder: **Götze, Otto Prof. Dr. med. Hygiene-Institut der Georg-August-Universität Göttingen Kreuzbergring 57 D-3400 Göttingen(DE)**

(72) Erfinder: **Götze, Otto, Prof. Dr. med. F.-v.-Bodelschwingh-Strasse 20 D-3400 Göttingen(DE)**
Erfinder: **Oppermann, Martin, Dr. med. Jüdenstrasse 8/9 D-3400 Göttingen(DE)**
Erfinder: **Schulze, Matthias, Dr. med. Theodor-Heuss-Ring 10 D-3000 Hannover 61(DE)**

(74) Vertreter: **Patentanwälte Grünecker, Kinkeldey, Stockmair & Partnerner Maximilianstrasse 58 D-8000 München 22(DE)**

(54) **Verfahren zum Nachweis und/oder der quantitativen Bestimmung von Komplementpeptid C5a und/oder C5adesarg.**

(57) Die Erfindung bezieht sich auf ein Verfahren zum Nachweis und/oder der quantitativen Bestimmung von Komplementpeptid C5a und/oder C5adesarg in biologischen Flüssigkeiten, das die folgenden Schritte umfaßt:

a) In-Berührung-bringen einer Probe der biologischen Flüssigkeit, die C5a und/oder C5adesarg enthält, mit einer Matrix, auf der eine erste Antikörper-Präparation (Akp 1), enthaltend Antikörper, die C5a und/oder C5adesarg binden, fixiert ist,

b) In-Berührung-bringen der aus a) resultierenden beladenen Matrix mit einer zweiten Antikörper-Präparation (Akp 2), enthaltend Antikörper und/oder Teile davon, die an das Komplementprotein C5 und/oder an C5a und/oder C5adesarg binden, wobei die gebundenen Antikörper und/oder Antikörperteile aus der zweiten Antikörper-Präparation detektierbar sind,

c) Detektieren der gebundenen Antikörper und/oder Antikörperteile aus der zweiten Antikörper-Präparation.

## VERFAHREN ZUM NACHWEIS UND/ODER DER QUANTITATIVEN BESTIMMUNG VON KOMPLEMENTPEPTID C5A UND/ODER C5ADESARG

Das Komplementpeptid C5a ist ein bei der proteolytischen Spaltung des Plasmaproteins C5 entstehendes Glykopeptid mit einem Molekulargewicht von 11300. C5a ist Bestandteil sowohl des klassischen als auch des alternativen Komplementaktivierungsweges und aktiviert alle Zellen, die C5a spezifische Membranrezeptoren an ihrer Oberfläche tragen, beispielsweise Granulozyten und Monozyten des menschlichen Blutes sowie Gewebemakrophagen. Das in vivo durch proteolytische Desarginierung aus C5a entstehende Komplementpeptid C5a-desarg 74, im folgenden kurz C5adesarg genannt, entfaltet hingegen keine oder eine nur stark verminderte biologische Wirkung, das heißt, die C5a spezifischen Wirkungen sind mit C5adesarg nicht mehr oder wesentlich schwächer nachweisbar.

C5a induzierte Wirkungen sind zum Beispiel eine in den aktivierten Zellen gesteigerte Adhärenz (zum Beispiel an Endothel-Zellen) und eine gerichtete Wanderung (Chemotaxis) zum Ort der höchsten Konzentration von C5a hin. C5a aktiviert den Sauerstoffmetabolismus der Zellen mit dem Ergebnis der Produktion und Freisetzung toxischer Sauerstoffmetaboliten und führt zur Freisetzung von biologisch aktiven Substanzen aus der Granula der Zellen. Zu diesen Substanzen gehören Proteasen, beispielsweise Kollagenase, Elastase und Kathepsine, die Gewebeproteine proteolytisch angreifen können, und weitere toxische Moleküle. Weiterhin kann es durch C5a-Wirkung über den Arachidonsäure-Stoffwechsel zur Bildung und Abgabe von Prostaglandinen, Leukotrienen und Thromboxanen kommen, die die verschiedensten Auswirkungen auf Zellen und Gewebefunktionen haben können (s. W. Vogt, 1986).

In einer Reihe von tierexperimentellen Untersuchungen und Beobachtungen an erwachsenen Patienten mit akutem Lungenversagen ("Adult Respiratory Distress Syndrom", ARDS) konnten Daten gewonnen werden, die für eine Beteiligung von C5a bei der Entstehung des ARDS sprechen. So wird beispielsweise in einer Publikation von Stevens et al. (1986) die Verwendung polyklonaler Antikörper gegen C5a/C5 in einem ARDS-Modell in Primaten beschrieben. Die mit den polyklonalen Antikörpern behandelten Tiere erkrankten weniger schwer, alle behandelten Tiere überlebten, während von vier nicht behandelten Tieren nur eines überlebte.

Die Erfinder der vorliegenden Erfindung konnten weiterhin einen monoklonalen Antikörper herstellen, der ebenfalls spezifisch mit C5/C5a bzw. C5adesarg reagiert. Einer dieser monoklonalen Antikörper, der mAK F24/1, ist für den Aufbau eines empfindlichen ELISA-Verfahrens zur Messung von C5a in Blut verwendet worden (Schulze und Götze, 1986). Der beschriebene Test erfordert jedoch die vollständige Ausfällung des Plasmaproteines C5 vor der Bestimmung des enthaltenen C5a bzw. C5adesarg.

In der EP 245 993 werden monoklonale Antikörper offenbart, die keine Spezifität für das Plasmaprotein C5 mehr aufweisen, sondern ausschließlich mit C5a und C5adesarg reagieren. In der Anmeldung wird die Möglichkeit der Verwendung dieser Antikörper für immunologische oder immundiagnostische Verfahren, auch für Sandwich-Immunassays, angedeutet. Die Anmeldung enthält jedoch keinen Hinweis auf die Verwendung von Antikörpern im Test, die mit dem nativen Plasmaprotein C5 reagieren, noch auf die Empfindlichkeit des verwendeten Verfahrens.

Aufgabe der Erfindung war es daher, ein Verfahren zur Verfügung zu stellen, mit dem der C5a- bzw. C5adesarg-Gehalt in biologischen Flüssigkeiten zuverlässig und empfindlich bestimmt werden kann.

Diese Aufgabe wird erfindungsgemäß durch ein Verfahren der eingangs genannten Art gelöst, das die folgenden Schritte umfaßt:

a) In-Berührung-bringen einer Probe, die C5a und/oder C5adesarg enthält, mit einer Matrix, auf der eine erste Antikörper-Präparation (Akp 1), enthaltend Antikörper, die C5a und/oder C5adesarg binden, fixiert ist;

b) In-Berührung-bringen der aus a) resultierenden beladenen Matrix mit einer zweiten Antikörper-Präparation (Akp 2), enthaltend Antikörper und/oder Teile davon, die an natives Komplementprotein C5 und/oder an C5a und/oder C5adesarg binden, wobei die gebundenen Antikörper und/oder Antikörperteile aus der zweiten Antikörper-Präparation detektierbar sind,

c) Detektieren der gebundenen Antikörper und/oder Antikörperteile aus der zweiten Antikörper-Präparation.

Das erfindungsgemäße Verfahren zeichnet sich durch eine außerordentliche Empfindlichkeit für den Nachweis von C5a und/oder C5adesarg sowohl in artifiziellen Systemen als auch in biologischen Flüssigkeiten, insbesondere in Plasma und Serum, aus. Weiterhin hat es den Vorteil, daß für seine Durchführung nicht ausschließlich C5a bzw. C5adesarg-spezifische Antikörper von Nöten sind, sondern in der zweiten Antikörper-Präparation Antikörper bzw. Teile davon verwendet werden können, die neben den C5a- bzw. C5adesarg-spezifischen Epitopen das native Komplementprotein erkennen. Auf diese Weise ist sichergestellt, daß empfindliche Tests in großer Menge und von gleichbleibender Qualität bereitgestellt werden können.

Das erfindungsgemäße Verfahren setzt nicht notwendigerweise vollständige Antikörper in den beiden Antikörper-Präparationen Akp 1 und Akp 2 voraus. Während jedoch die an die Matrix gekoppelten Antikörper der Akp 1 in der Regel vollständige Immunglobuline sein werden, ist es möglich, in der Akp 2 die spezifisch mit C5a und/oder C5adesarg, gegebenenfalls auch mit C5 reagierenden Bestandteile in Form von Antikörper-Fragmenten, beispielsweise Fab- oder F(ab')$_2$-Fragmenten, einzusetzen. Die Wahl der jeweils verwendeten Akp 2 hängt nicht zuletzt von der Spezifität und Quantität der zur Verfügung stehenden Antikörper für die Akp 2 ab.

In einer bevorzugten Ausführungsform enthalten die Antikörper-Präparationen Akp 1 und/oder Akp 2 monoklonale Antikörper bzw. Antikörper-Fragmente, wobei diese bevorzugt aus Ratten, Kaninchen, Schweinen oder Affen gewonnen werden. Besonders bevorzugt sind solche humanen oder murinen Ursprungs. Die Erzeugung von monoklonalen Antikörpern gewünschter Spezifität in der Maus wird durch die für diesen Organismus bereitstehende Technologie sowie die leichte Verfügbarkeit und Züchtbarkeit von Mäusen gefördert. Die Bereitstellung monoklonaler Antikörper humanen Ursprungs hat den wesentlichen Vorteil, daß diese bei einer eventuellen Applikation am Menschen weniger leicht eine Immunantwort hervorrufen, als das beispielsweise mit murinen Antikörpern der Fall wäre.

Erfindungsgemäß binden die monoklonalen Antikörper bzw. Antikörperteile der ersten oder zweiten Antikörperpräparation an eine Rezeptorbindungsstelle für den C5a-spezifischen Rezeptor im Komplementprotein C5a und/oder C5adesarg, während sie nicht an die mit der Rezeptorbindungsstelle übereinstimmende Aminosäuresequenz im nativen C5-Molekül binden. Bei der genannten Rezeptorbindungsstelle handelt es sich offensichtlich um ein Neoepitop auf dem Peptid, das auf dem Vorläuferprotein C5 der Antikörpererkennung nicht zugänglich ist. Ein solcher Rezeptorbindungsstellen-spezifischer monoklonaler Antikörper wird mit der vorliegenden Erfindung erstmals zur Verfügung gestellt.

In einer bevorzugten Ausführungsform enthält die erste Antikörper-Präparation (Akp 1) den monoklonalen Antikörper C17/5 (CNCM I-887) und/oder funktionell äquivalente Antikörper. C17/5 reagiert spezifisch mit C5a/C5adesarg, jedoch nicht mit dem nativen Plasmaprotein C5. Umfangreiche Untersuchungen haben ergeben, daß es sich bei dem von C17/5 erkannten Neoepitop um die Rezeptorbindungsstelle des C5a-spezifischen Rezeptors handelte. Die Verwendung dieses für die Spaltprodukte von C5 spezifischen monoklonalen Antikörpers macht die mit vielen Problemen verbundene Präzipitation im Serum vorhandener C5-Anteile vor der Durchführung der Messung überflüssig.

Erfindungsgemäß wird C17/5 zur Durchführung des Verfahrens an eine Matrix gekoppelt zur Verfügung gestellt. Der solchermaßen insolubilisierte Antikörper entzieht der zu untersuchenden Probe lediglich C5a- und C5adesarg-Moleküle, jedoch nicht in der Probe befindliches C5. Die Kopplung an die jeweilige Matrix, z. B. Mikrotiterplatten, Sepharosepartikel oder andere im Stand der Technik bekannte Träger, erfolgt nach dem Fachmann geläufigen Verfahren.

Die zweite Antikörper-Präparation (Akp 2) enthält bevorzugt den monoklonalen Antikörper G25/2 (CNCM I-889) und/oder Teile davon und/oder funktionell äquivalente Antikörper. G25/2 ist ein monoklonaler Antikörper, der ein Epitop erkennt, das gleichermaßen im Komplementprotein C5 und in dessen Spaltprodukten C5a und C5adesarg vorhanden ist. G25/2 bindet daher an eine andere Stelle des C5a-Moleküls als C17/5; seine Bindung interferiert nach den im folgenden beschriebenen Ergebnissen nicht mit der Bindung von C17/5. Im erfindungsgemäßen Verfahren kommt der zweiten Antikörper-Präparation die Aufgabe zu, an die bereits aus der Probe durch Reaktion mit der ersten Antikörper-Präparation angereicherten C5a- bzw. C5adesarg-Moleküle zu binden und so ihren Nachweis zu ermöglichen.

Für den Nachweis gebundener Antikörper-Moleküle bzw. Antikörper-Fragmente aus der zweiten Antikörper-Präparation bestehen verschiedene Möglichkeiten. Allgemein kann zwischen einer direkten Detektierbarkeit und einer indirekten Detektierbarkeit der gebundenen Moleküle aus der zweiten Antikörper-Präparation unterschieden werden. Eine direkte Detektion kann beispielsweise durch die Kopplung von radioaktiven, fluoreszierenden oder chemilumineszierenden Molekülen erzielt werden. Ein gebräuchliches Verfahren für eine radioaktive Markierung ist beispielsweise die Jodierung von Antikörpern bzw. Antikörper-Fragmenten mit $^{125}J$, wobei die Menge der gebundenen Radioaktivität entweder autoradiographisch oder durch Scintillationsmessung bestimmt wird. Da der Umgang mit radioaktiven Materialien zunehmend weniger gewünscht wird, erfährt die Verwendung von Fluoreszenz-Markierungen eine zunehmende Bedeutung. Ein gebräuchliches Verfahren ist in diesem Zusammenhang die Umsetzung von Fluoresceinisothiocyanat mit einem geeigneten Antikörper, der Bestandteil der Akp 2 werden soll. Weitere alternative Verfahren sehen die Kopplung von chemilumineszierenden Molekülen vor.

Im Fall der direkten Detektierbarkeit des Antikörpers bzw. Antikörper-Fragmentes bedarf es nach der Umsetzung mit der beladenen, bereits mit der Probe vorinkubierten Matrix keiner weiteren Folgereaktionen. Der Nachteil dieser Methoden besteht jedoch in einer oft geringen Halbwertszeit bzw. Haltbarkeit der gekoppelten Marker. Eine Alternative zu direkt detektierbaren Antikörpern bzw. Antikörper-Fragmente bieten indirekt detektierbare Antikörper bzw. Antikörper-Fragmente. In diesem Fall werden die in Frage stehenden Antikörper, die Bestandteil der Akp 2 sind, vorzugsweise mit einem Markerenzym, beispielsweise alkalischer Phosphatase, $\beta$-Galaktosidase, Mikroperoxidase oder Meerrettichperoxidase gekoppelt, dessen enzymatische Aktivität in einer weiteren Inkubation zu physikalisch meßbaren Veränderungen führt. Die genannten vier Enzyme sind beispielsweise unter geeigneten Bedingungen in der Lage, durch eine enzymatische Reaktion Farbreaktionen hervorzurufen. Die optisch meßbare Menge der enzymatisch hergestellten Farbe ist dabei ein Maß für die Menge an gebundenem, mit dem jeweiligen Enzym gekoppeltem Antikörper. Diese wie auch andere Markerenzyme müßten nicht unbedingt kovalent an den jeweils in der Akp 2 enthaltenen Antikörper bzw. das entsprechende Antikörper-Fragment gebunden sein, sondern können ebenso in Form eines Enzym-Anti-Enzym-Immunkomplexes vorliegen. Diese Enzym-Anti-Enzym-Immunkomplexe werden normalerweise vorgeformt, bevor sie zum zur Reaktion vorgesehenen Antikörper zugesetzt werden.

Eine weitere Möglichkeit des indirekten Nachweises gebundener Antikörper bzw. Antikörper-Fragmente aus der zweiten Antikörper-Präparation bietet deren Biotinylierung vor der Reaktion mit der bereits probenbeladenen Matrix. Gebundene biotinylierte Antikörper bzw. Antikörper-Fragmente werden durch Umsetzung mit Avidin oder Streptavidin nachgewiesen, an das wiederum ein enzymatisch aktives Markerenzym, beispielsweise eines der oben genannten Markerenzyme, gekoppelt ist.

Eine weitere Möglichkeit der Detektion der Antikörper bzw. Antikörperteile aus der Akp 2 besteht in deren Nachweis durch Antikörper einer dritten Antikörperpräparation. Voraussetzung für diese Verfahrensweise ist, daß die Antikörper bzw. Antikörperteile aus der Akp 1 und der Akp 2 aus verschiedenen Säugetierspecies stammen, um so die Immunglobuline der Akp 2 spezifisch nachweisen zu können. Handelt es sich beispielsweise bei den Antikörpern der Akp 1 um Mäuseantikörper, bei den Antikörpern der Akp 2 um Humanantikörper, so können diese Antikörper der Akp 2 durch geeignet markierte Ziege-Anti-Mensch-Immunglobuline nachgewiesen werden. Für die Detektion kann dabei jedes der bereits weiter oben beschriebenen Verfahren verwendet werden. Auf dem Markt sind eine Vielzahl von gegen den $F_c$-Anteil der Immunglobuline aus vielerlei Organismen spezifischen markierten Immunglobulinen erhältlich.

Das erfindungsgemäße Verfahren ermöglicht den Nachweis von C5a bzw. C5adesarg bis hin zu einer Konzentration von 20 pg/ml; damit ist ein wesentlicher Fortschritt hinsichtlich der Empfindlichkeit im Vergleich zu bekannten Verfahren erzielt. So liegt beispielsweise die Empfindlichkeit eines verwandten Testes, nämlich des von Takeda et al. (1987) beschriebenen Radioimmunassays für C5adesarg, bei 1 ng/ml und damit um den Faktor 50 niedriger. Die mit dem erfindungsgemäßen Test erreichbare Empfindlichkeit ermöglichte es erstmals, die Plasmaspiegel von C5a in EDTA-Plasma normaler Probanden festzustelllen.

Die oben erwähnte Aufgabe wird weiterhin gelöst durch einen Testkit für die Durchführung des oben beschriebenen Verfahrens, wobei dieser Testkit die folgenden Bestandteile umfaßt:

a) Eine erste Antikörper-Präparation (Akp 1), enthaltend Antikörper, die an C5a und/oder C5adesarg binden,

b) eine zweite Antikörper-Präparation (Akp 2), enthaltend Antikörper und/oder Teile davon, die an natives Komplementprotein C5 und/oder C5a und/oder C5adesarg binden, wobei die gebundenen Antikörper und/oder Antikörperteile aus der zweiten Antikörper-Präparation detektierbar sind,

c) gegebenenfalls ein oder mehrere Nachweisreagenzien, enthaltend Enzyme und/oder Substrate, und/oder eine dritte Antikörperpräparation für die Detektionsreaktionen.

Mit dem erfindungsgemäßen Testkit sollen dem untersuchenden Arzt oder Wissenschaftler alle Möglichkeiten an die Hand gegeben werden, die C5a- bzw. C5adesarg-Konzentration der jeweils interessierenden biologischen Flüssigkeit, z.B. Blutplasma oder -serum, aber auch Urin, Speichel, Schweiß oder Tränenflüssigkeit, sofort bestimmen zu können. Der Testkit enthält eine erste Antikörper-Präparation, enthaltend Antikörper, die an C5a und/oder C5adesarg binden. Gegebenenfalls kann diese Antikörper-Präparation bereits an eine Matrix gekoppelt zur Verfügung gestellt werden, beispielsweise in Form vorpräparierter Mikrotiterplatten. In Abhängigkeit von den jeweiligen Bedürfnissen können die Antikörper jedoch an jede gebräuchliche feste Matrix, beispielsweise Plastikröhrchen, Polystyrolperlen, Sepharosepartikel oder ähnliches gekoppelt werden. Der Testkit umfaßt weiterhin eine zweite Antikörper-Präparation, wobei die darin enthaltenen Antikörper bzw. Antikörper-Fragmente detektierbar sein sollten. Für die Detektion eröffnen sich die gleichen Möglichkeiten, wie sie oben für das Verfahren bereits beschrieben worden sind. Im Fall von indirekt detektierbaren Antikörpern bzw. Antikörper-Fragmenten aus der zweiten Antikörper-Präparation kann der Testkit weiterhin eine oder mehrere Nachweisreagenzien enthalten. Unter Nachweisreagenzien sind in diesem Zusammenhang entweder an spezifisch reagierende Moleküle gekoppelte Enzyme zu verstehen, beispielsweise Avidin gekoppelte $\beta$-Galaktosidase, und/oder Substrate für das verwendete Markerenzym oder eine dritte Antikörperpräparation, deren Antikörper spezifisch mit denen der zweiten Antikörperpräparation reagieren.

In bevorzugten Ausführungsformen umfaßt sowohl die erste Antikörper-Präparation als auch gegebenenfalls die zweite Antikörper-Präparation monoklonale Antikörper bzw. Antikörperteile. Hier wie auch im oben beschriebenen Verfahren ist die Verwendung von Antikörpern aus jedem Säugerorganismus, beispielsweise Primaten-, Schweine-, Kaninchen- oder Ratten-Antikörper, möglich. Bevorzugte Ausführungsformen betreffen Maus-oder Mensch-Antikörper. Für die Zwecke des erfindungsgemäßen Testkits spielt es dabei keine Rolle, welchen Isotyps der jeweils verwendete Antikörper ist. Die Verwendung von IgG-Antikörpern ist bevorzugt, die von IgM-Antikörpern jedoch genauso möglich.

Der in der Akp 1 oder Akp 2 enthaltene Antikörper, der spezifisch mit C5a und/oder C5adesarg reagiert, jedoch keiner Bindung an das Muttermolekül C5 unterliegt, ist in einer besonders bevorzugten Ausführungsform ein Antikörper, der mit der erst in den Spaltprodukten von C5 freigelegten Rezeptorbindungsstelle für den C5a-spezifischen Rezeptor reagiert.

Die erste Antikörper-Präparation des erfindungsgemäßen Testkits umfaßt bevorzugt den monoklonalen Antikörper C17/5 und/oder funktionell äquivalente Antikörper. Unter funktionell äquivalenten Antikörpern soll in diesem Zusammenhang ein Antikörper verstanden werden, der auf dem nativen Komplementprotein C5 keine Erkennungsstelle findet, sondern ausschließlich und spezifisch mit C5a bzw. C5adesarg reagiert. Im Gegensatz dazu reagiert der für die zweite Antikörper-Präparation bevorzugte Antikörper, der monoklonale Antikörper G25/2 (CNCM I-889) und/oder jeder funktionell äquivalente Antikörper, nicht nur mit Epitopen von C5a und/oder C5adesarg, sondern ebenso mit einem bereits auf C5 nachweisbaren Epitop. In der Praxis spielt die Bindungsfähigkeit von G25/2 an C5 keinerlei Rolle, da durch den vorhergegangenen Schritt der Inkubation der mit einem spezifisch mit C5a bzw. C5adesarg reagierenden Antikörper beladenen Matrix mit der entsprechenden Probe C5-Moleküle bereits eliminiert worden sind.

Die mit dem erfindungsgemäßen Testkit zur Verfügung gestellten Antikörper bzw. Antikörper-Fragmente der zweiten Antikörper-Präparation sind entweder direkt oder indirekt detektierbar. Die verschiedenen Möglichkeiten einer Markierung, die zu einer direkten Detektierbarkeit führt, sowie indirekter Markierungen sind bereits oben diskutiert. Auch für den erfindungsgemäßen Testkit sieht eine bevorzugte Ausführungsform die Verwendung von alkalischer Phosphatase, $\beta$-Galaktosidase, Mikroperoxidase oder Meerrettichperoxidase als gekoppeltes Markerenzym vor.

Anstelle einer direkten Enzymmarkierung der Antikörper bzw. Antikörper-Fragmente der zweiten Antikörper-Präparation ist die Bereitstellung Biotinmarkierter Antikörper bzw. Antikörper-Teile möglich. In diesem Fall wird darüberhinaus ein modifiziertes, bevorzugt mit einem Markerenzym gekoppeltes Avidin- oder Streptavidin-Präparat sowie das entsprechende Substrat für das Enzym bereitgestellt.

Weiterhin ist es möglich, den bereits vorgeformten Antikörper-C5a/C5adesarg-Antikörper (Antikörper-Fragment)-Komplex durch Reaktion mit markierten Antikörpern, die spezifisch mit dem Antikörper bzw. Antikörper-Fragment aus der zweiten Antikörper-Präparation reagieren, umzusetzen. Dies setzt allerdings voraus, daß die $F_c$-Anteile der Antikörper der zweiten Antikörper-Präparation andere Eigenschaften aufweisen, als die der ersten, das heißt, daß die Antikörper der ersten und zweiten Präparation aus verschiedenen Organismen gewonnen worden sein müssen.

Gegenstand der vorliegenden Erfindung sind weiterhin die Zellinien, die die in dem erfindungsgemäßen Verfahren verwendeten monoklonalen Antikörper sezernieren. Dabei handelt es sich um Zellinien, die einen monoklonalen Antikörper ausscheiden, der im Komplementprotein C5a und/oder C5adesarg an die Rezeptorbindungsstelle für den C5a-spezifischen Rezeptor, jedoch nicht an die korrespondierende Amminosäuresequenz im nativen C5-Molekül bindet. Zellinien, die Antikörper mit solchen Eigenschaften sezernieren, sind trotz des großen Wertes solcher Antikörper für die klinische Anwendung und die medizinische Forschung bisher nicht beschrieben. Die Spezifität der sezernierten Antikörper für die Rezeptorbindungsstelle läßt sich, wie in den Figuren und den Beispielen näher erläutert wird, schlüssig durch Experimente mit anti-idiotypischen Antikörpern nachweisen.

Die Erfindung betrifft weiterhin die Zellinien, die die beiden bevorzugt zu verwendenden Antikörper, nämlich C17/5 und G25/2, sezernieren. Bei beiden Zellinien handelt es sich um Hybridomas, deren Herstellung in den nachfolgenden Beispielen ausführlich beschrieben wird. Die den monoklonalen Antikörper C17/5 sezernierende Zellinie ist bei der CNCM mit der Hinterlegungsnummer I-887 hinterlegt worden, die den Antikörper G25/2 sezernierende Zellinie ebenfalls bei der CNCM mit der Hinterlegungsnummer I-889.

Die von den erwähnten Zellinien produzierten monoklonalen Antikörper sind ebenfalls Gegenstand dieser Erfindung.

Zusammensetzungen, die einen monoklonalen Antikörper nach mindestens einem der Ansprüche 25 bis 30 enthalten, sind nicht nur für die Verwendung in den oben beschriebenen Testkits geeignet, sondern können darüberhinaus klinische Bedeutung bei der Verringerung der Konzentration aktiven C5a's im Blut eines Patienten gewinnen. Die Erfindung bezieht sich daher weiterhin auf die die genannten Antikörper enthaltenden Zusammensetzungen sowie die Verwendung der monoklonalen Antikörper zur Behandlung von Erkrankungen, die durch C5a-Aktivität hervorgerufen werden. In diesem Zusammenhang soll besonders die Verwendung der erfindungsgemäßen monoklonalen Antikörper zur Therapie und/oder Prophylaxe von ARDS genannt werden. Weiterhin ist die Verwendung dieser Antikörper bei intra- oder extravasculärer Aktivierung des Komplementsystems mit möglicherweise pathologischen Auswirkungen, z.B. bei Schock, Sepsis und anderen akut oder chronisch verlaufenden Erkrankungen, die mit einer Aktivierung des Komplementsystems einhergehen, sowie bei entzündlich-immunologischen Erkrankungen, z.B. bei rheumatischer Polyarthritis und Lupus erythematodes, vorgesehen.

Gegenstand der Erfindung ist weiterhin ein Verfahren zum Herstellen von Hybridoma-Zellinien, die ein nach proteolytischer Spaltung von C5 auf C5a und/oder C5adesarg exponiertes Neoepitop erkennen, umfassend die folgenden Schritte:

a) Inkubieren von C5a mit einem gegebenenfalls an einen Träger gekoppelten Antikörper, dessen Bindung an C5a C5a-spezifische Aktivitäten nicht oder nur geringfügig beeinflußt,

b) gegebenenfalls Inkubieren des an den Antikörper gebundenen C5a's mit Zellen, die C5a-Rezeptoren aufweisen, Anreichern der Antikörper-C5a-Zellaggregate, Lysieren der Zellen, Wiedergewinnen der Antikörper-C5a-Komplexe, bevorzugt durch Dichtegradienten-Zentrifugation,

c) Immunisieren eines Säugetieres mit den gegebenenfalls angereicherten Antikörper-C5a-Komplexen,

d) Gewinnen von Antikörper-produzierenden Zellen aus dem immunisierten Säugetier, bevorzugt von Milzzellen, und Immortalisieren dieser Zellen in an sich bekannter Weise.

Das erfindungsgemäße Verfahren führt mit hoher Ausbeute zu Hybridoma-Zellinien mit den eingangs erwähnten Eigenschaften.

Gegenstand der Erfindung ist weiterhin ein monoklonaler Antikörper, der ein anti-idiotypischer Antikörper gegen Antikörper ist, die spezifisch mit der Rezeptorbindungsstelle für den C5a-spezifischen Rezeptor auf C5a bzw. C5adesarg reagieren. Ein solcher anti-idiotypischer Antikörper bindet an Blutmonozyten und Granulozyten in fast identischer Weise wie das Komplementprotein C5a selber. Die Bindung des anti-idiotypischen Antikörpers an Blutmonozyten oder Granulozyten läßt sich jedoch verhindern, wenn diese zuvor mit C5a abgesättigt werden.

Auch für diesen monoklonalen Antikörper ist es bevorzugt, daß seine variable und/oder seine konstante Region aus einem Säugetier stammt, bevorzugt aus Ratte, Kaninchen, Schwein oder Affe, besonders bevorzugt aus Maus oder Mensch. Für die therapeutische Anwendung solcher Antikörper sollte bevorzugt zumindest die konstante Region humanen Ursprungs sein. Die erfindungsgemäßen anti-idiotypischen Antikörper können sowohl vom IgM- als auch vom IgG-Typus sein.

Ein bevorzugter anti-idiotypischer monoklonaler Antikörper ist der von der Zellinie F23/14, die bei der CNCM mit der Hinterlegungsnummer I-888 hinterlegt ist, sezernierte Antikörper. Es handelt sich dabei um einen Antikörper der Klasse IgM/Kappa (IgM, K). Die diesen anti-idiotypischen Antikörper gegen C17/5 sezernierende Zellinie wurde hergestellt, indem BALB/c Mäuse, die auch für die Herstellung von C17/5 verwendet worden waren (s. Beispiel 1), mit bestrahlten Hybridoma-Zellen der Linie C17/5 immunisiert wurden. Die Immunisierung, Fusionierung und Klonierung wurden nach dem Fachmann bekannten Verfahren durchgeführt (siehe Peters et al., 1985). Die zur Immunisierung verwendeten Hybridoma-Zellen sind dabei in diesem speziellen Fall mit 2000 rad bestrahlt worden.

Der erfindungsgemäße monoklonale Antikörper F23/14 kann dazu verwendet werden, die Aktivität von C5a, das mit dem C5a-Rezeptor reagiert, durch Blockierung des Rezeptors spezifisch zu inhibieren. F23/14 imitiert die Bindungseigenschaften von C5a erfolgreich.

Die folgenden Figuren und Beispiele erläutern die Erfindung.

Figurenbeschreibung

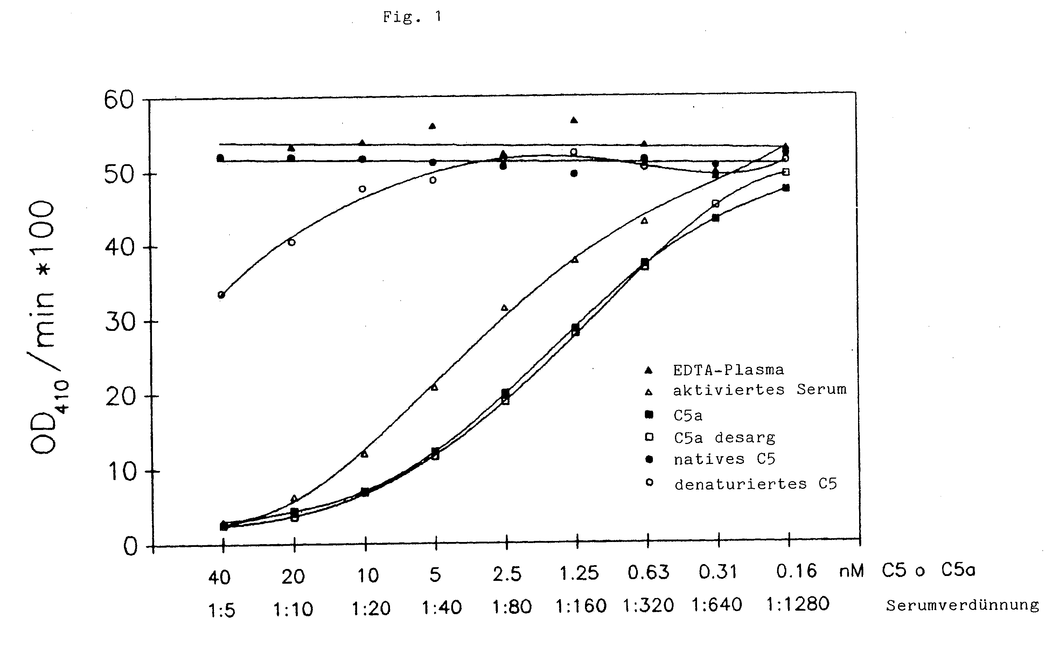

Fig. 1 zeigt die Ergebnisse eines für EDTA-Plasma, aktiviertes Serum, C5a, C5adesarg, natives und denaturiertes C5 durchgeführten kompetitiven ELISA-Verfahrens unter Verwendung des mAK C17/5. C5a und C5adesarg können mit identischer Empfindlichkeit (identischer Kurvenverlauf) gemessen werden. Natives isoliertes C5 wird in diesem Test nicht erfaßt, ebensowenig wie C5 in humanem EDTA-Plasma. Denaturiertes C5 wird in dem Test mit geringer Empfindlichkeit erfaßt (es handelt sich um isoliertes C5, das in-vitro im Labor denaturiert wurde). C5a/C5adesarg in Komplement-aktiviertem Humanserum wird ebenfalls in einer Dosis-abhängigen Weise mit diesem Test quantifiziert.

In die Näpfe von Mikrotiterplatten, in die der mAK C17/5 adsorbiert wurde (Beschichtung der Platten mit 0,1 ml einer mAK Konzentration von 40 ug/ml) wurden die auf der Abszisse angegebenen Mengen/Konzentrationen von C5, C5a oder Serum in Gegenwart von Biotin-konjugiertem, isoliertem C5a in 0,1 ml Volumen zugesetzt. Nach einer Inkubation von 1 Stunde (bei 22°C) und nach einem Waschschritt wurde das gebundene Biotin-C5a mit Hilfe eines Streptavidin-Peroxidase-Komplexes über die enzymatische Peroxidasereaktion nachgewiesen. Auf der Ordinate ist die Enzymaktivität (Erhöhung der OD bei 410 nm pro Minute) aufgetragen. Es handelt sich also um einen kompetitiven ELISA, in dem die Verhinderung der Bindung von Biotin-C5a an den Festphasen-gebundenen mAK C17/5 durch die gleichzeitige Inkubation mit kompetierenden Antigenen geprüft wird. Wie aus der Abbildung hervorgeht, kompetieren natives, isoliertes C5 und C5 in EDTA-Plasma mit C5a um die Bindungsstelle am mAK C17/5 nicht. Nur partielle Kompetition, bei hohen Konzentrationen, zeigt sich mit "denaturiertem C5" (10 mal aufgetaut und eingefroren). Typische Kompetitionskurven ergeben sich für C5a und C5adesarg, die beide identisch verlaufen, und für C5a, das in Komplement-aktiviertem Serum vorhanden ist.

Die Bindung von 2 ng C5a-Biotin an den mAK C17/5 (Einstellung des Systems auf 75% Sättigung) wird in Gegenwart von etwa 2 nM C5a zu 50% inhibiert. In Gegenwart von 20 nM C5a ist die Inhibition fast komplett. Dagegen zeigen 40 nM C5 oder 2 ug C5 in Serum/Plasma (d.h. 0,1 ml 1:5 verdünntes Serum/Plasma) keine inhibitorische Wirkung. Das heißt, daß isoliertes C5 in mehr als 20fachem molaren Überschuß nicht inhibiert und daß C5 in Serum/Plasma in sogar 1000fachem Gewichtsüberschuß (d.h. 58fachem molaren Überschuß) ebenfalls nicht inhibiert. In der Fig. 1 verlaufen deshalb die beiden Kurven parallel zur Grundlinie.

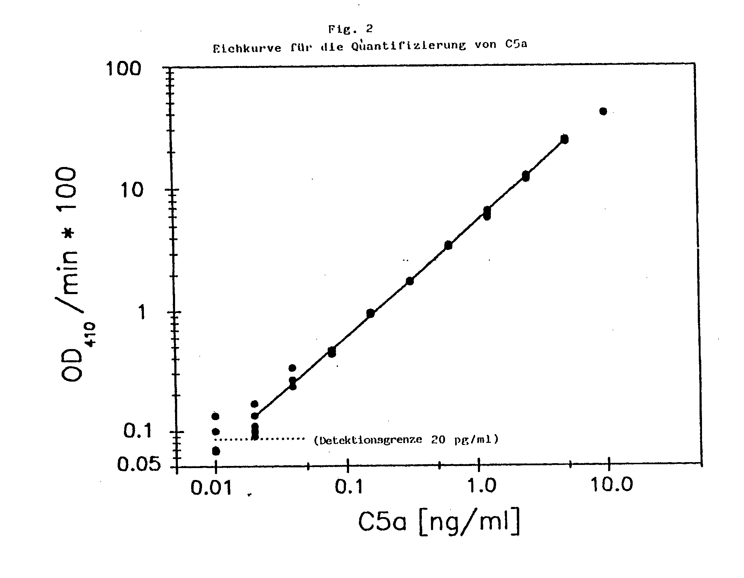

Fig. 2 gibt die Eichkurve für die Quantifizierung von C5a/C5adesarg wieder. Die Nachweisgrenze für die beiden Peptide beträgt 20 pg/ml, d.h., absolut beträgt sie 2 pg pro Testansatz, da jeweils 0,1 ml in die ELISA-Platten eingesetzt wird. Die Nachweisgrenze ist bestimmt als Mittelwert von 8 Kontrollansätzen ($\bar{x}_0$ 2,6 S.D.).

Die ELISA-Platten werden mit 0,1 ml C17/5-Antikörperlösung der Konzentration 40 ug/ml in 50 mM Na-Carbonat, pH 10,6, beschichtet. Nach 2 mal Waschen (je 0,250 ml PBS, enthaltend 20 mM EDTA, 0,05% (Vol/Vol) Tween 20 und 1% (Gew./Vol) Gelatine) erfolgt die Inkubation der Platten mit rekombinantem C5a in den auf der Abszisse angegebenen Konzentrationen (22°C, 2 Stunden). Danach wird wieder mit 0,25 ml PBS-EDTA-Tween 20 gewaschen. Es folgt dann eine Inkubation mit biotinyliertem mAK G25/2 (1 ug/PBS-EDTA-Tween 20) und, nach 4 mal Waschen mit PBS-EDTA-Tween 20 (wie oben), die Inkubation mit Streptavidin-Peroxidasekonjugat (ABTS von Amersham, Braunschweig, 1:1000 in PBS-EDTA-Tween 20 verdünnt). Daran schließt sich der Nachweis der Peroxidase-Aktivität im Photometer mit Hilfe des Substrates ABTS (von Boehringer Mannheim) an. Die Aktivität ist auf der Ordinate aufgetragen (Zunahme der OD bei 410 nm pro Minute).

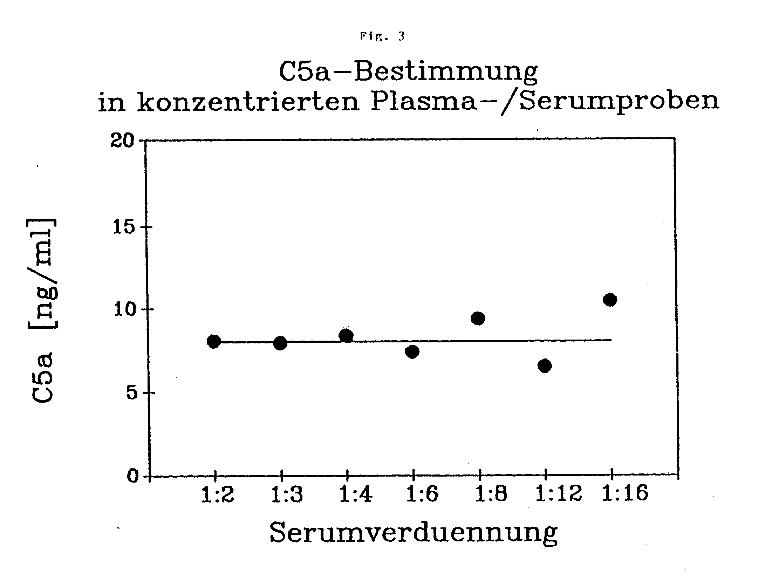

Fig. 3 zeigt eine C5a/C5adesarg-Bestimmung aus konzentrierten Plasma- bzw. Serumproben bei Verdünnungen der Proben von 1:2 oder höher. Störende Faktoren sind offenbar auch in 1:2 verdünntem Plasma nicht vorhanden.

Das ELISA-Verfahren wurde wie für Fig. 2 beschrieben durchgeführt. Es wurden Plasma- bzw. Serumproben verwendet, zu denen 8 ng C5a zugegeben wurden. Das angewendete ELISA-Verfahren wird durch verschiedene Plasma- bzw. Serumkonzentrationen nicht beeinflußt. Sogar bei einer Plasma- bzw. Serumverdünnung von nur 1:2 sind keine Störfaktoren erkennbar.

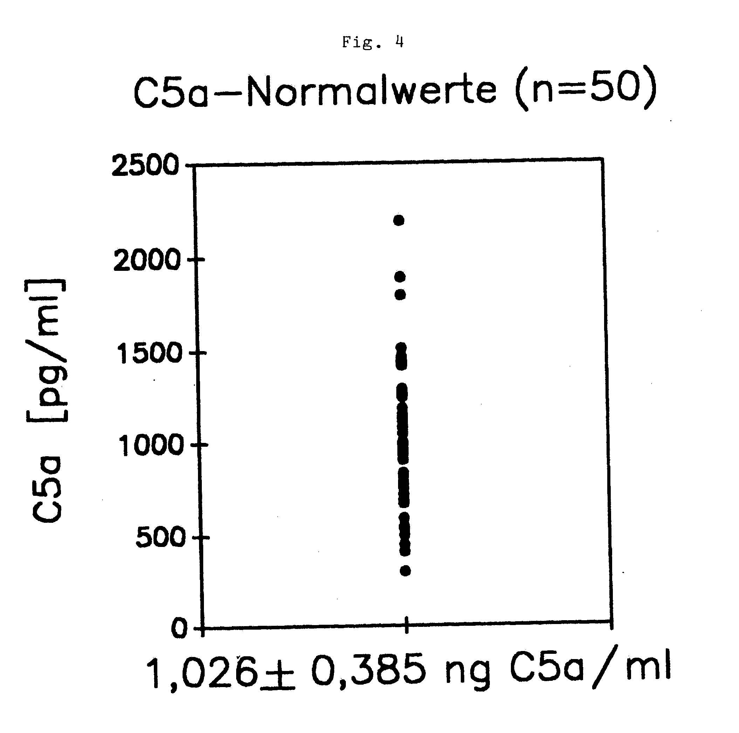

Fig. 4 gibt die Ergebnisse der Bestimmung der Konzentrationen von C5a/C5adesarg in EDTA-Plasma von normalen Probanden in 50 Proben wieder.

Auf der Ordinate ist der C5a-Gehalt der einzelnen untersuchten Proben ablesbar (jeder Punkt bedeutet den Untersuchungswert eines bestimmten Plasmas in pg pro ml). Er lag zwischen 0,298 und 2,192 ng/ml. Anstelle einer Abszisse ist der Mittelwert der an 50 Proben gemessenen C5a-Konzentrationen im EDTA-Plasma angegeben. Er beträgt 1,026 ng C5a/ml Plasma +/- 0,385 (1 SD).

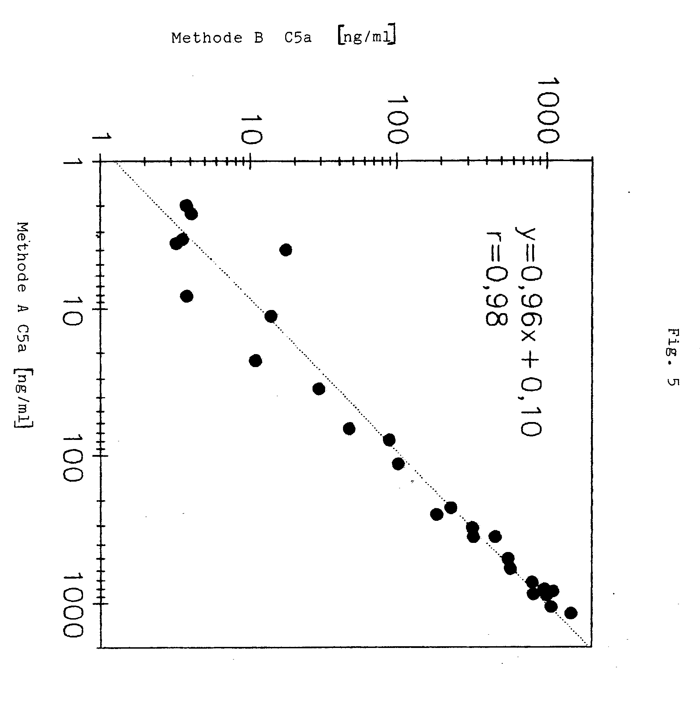

Fig. 5 zeigt eine Korrelation zwischen dem alten Test von Schulze und Götze und dem neuen Test, der den Neoepitop-spezifischen mAK C17/5 verwendet. Der neue Test ist unter "Methode A" aufgeführt, der alte Test unter "Methode B". Bei den verwendeten Proben handelt es sich um Zymosan-aktivierte humane Serumproben, die je nach Inkubationszeit bzw. Zymosan-Dosierung verschieden hohe Mengen von C5a/C5adesarg enthalten. Die Ergebnisse beider Verfahren sind hochkorreliert (r = 0,98). Beide Verfahren wurden an denselben Proben durchgeführt.

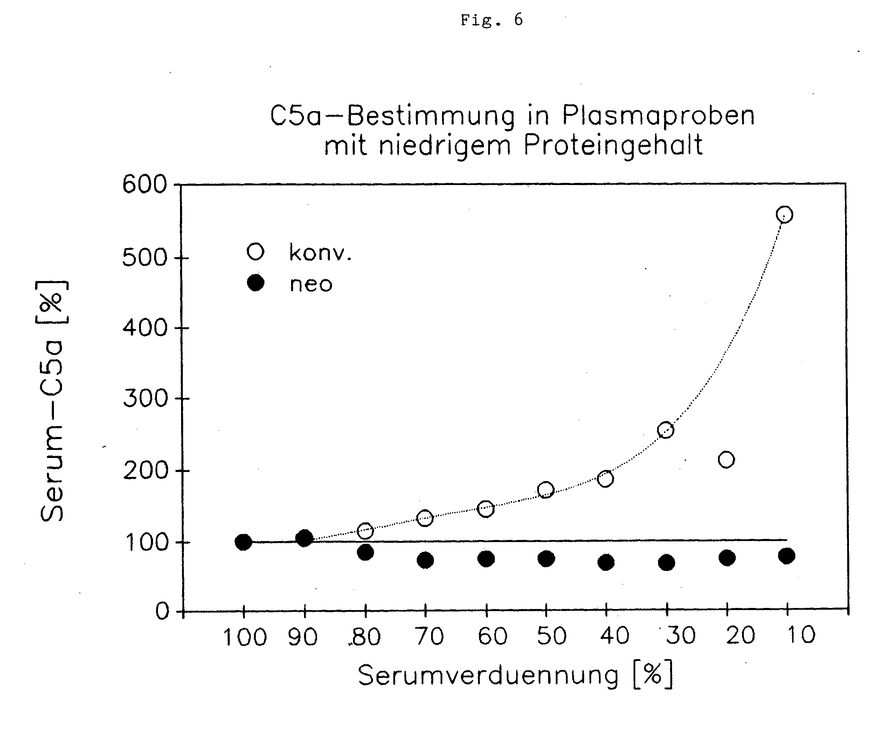

Fig. 6 illustriert die Zuverlässigkeit der C5a-Bestimmung nach dem erfindungsgemäßen Verfahren auch in Plasma- bzw. Serumproben mit erniedrigtem Proteingehalt. Das neue ELISA-Verfahren (Verwendung des mAK C17/5) wird durch die Verdünnung des Serums nicht beeinflußt (Kurve "neo"). Bei den bisher angewendeten Verfahren (Kurve "konv"), bei dem das Muttermolekül C5 durch einen Präzipitationsschritt entfernt werden mußte, ist die Serumverdünnung (d.h. die Proteinkonzentration) von entscheidender Bedeutung. Bei höheren Serumverdünnungen wird nicht das gesamte C5 präzipitiert; ein Signal im ELISA beruht dann nur zum Teil auf der Anwesenheit von C5a, zu einem größeren Teil aber auf der Anwesenheit von C5, das nicht vollständig entfernt wurde. Dies ist ein für die klinische Anwendung besonders wichtiger Punkt, da die klinischen Plasmaproben sich im Proteingehalt erheblich unterscheiden können. Praktisch alle Präzipitationsverfahren sind jedoch abhängig von der Gesamtproteinkonzentration der Proteinlösungen, die dem Präzipitationsverfahren unterworfen werden. Bei einer Proteinkonzentrationsverminderung von nur 20% wird ein ELISA-Verfahren, das einen Präzipitationsschritt voraussetzt, schon falsch hohe Werte liefern, weil aus diesen Proben C5 nicht vollständig präzipitiert werden kann.

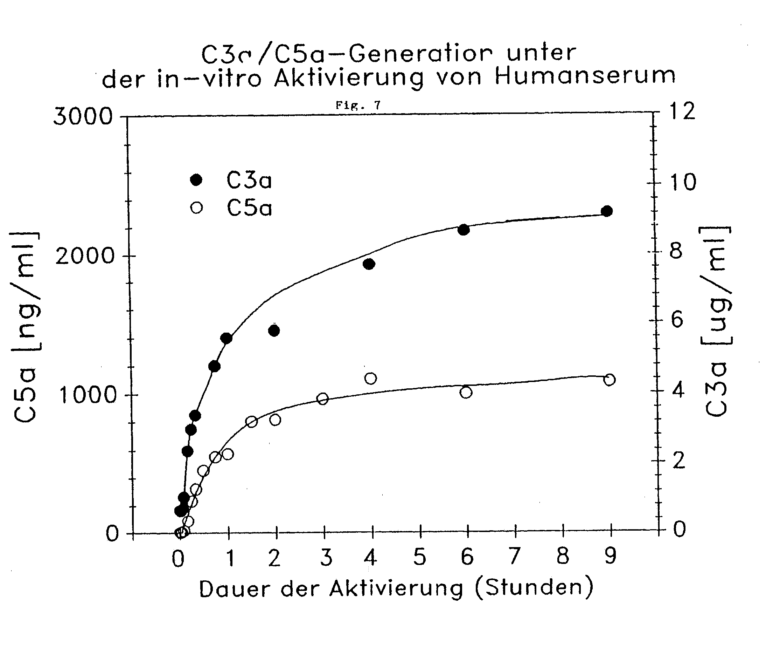

Fig. 7 zeigt die Kinetik der C5a-Entstehung (in ng/ml), die durch die Zugabe von Zymosan (Aktivator der alternativen Komplementaktivierung) bei 37° C erfolgt. Zusätzlich aufgetragen auf der zweiten Ordinate ist die Entstehung von C3a (in ug/ml). Die Abbildung zeigt, daß mit Hilfe des neuen Verfahrens die Generation von C5a im Humanserum quantitativ verfolgt werden kann.

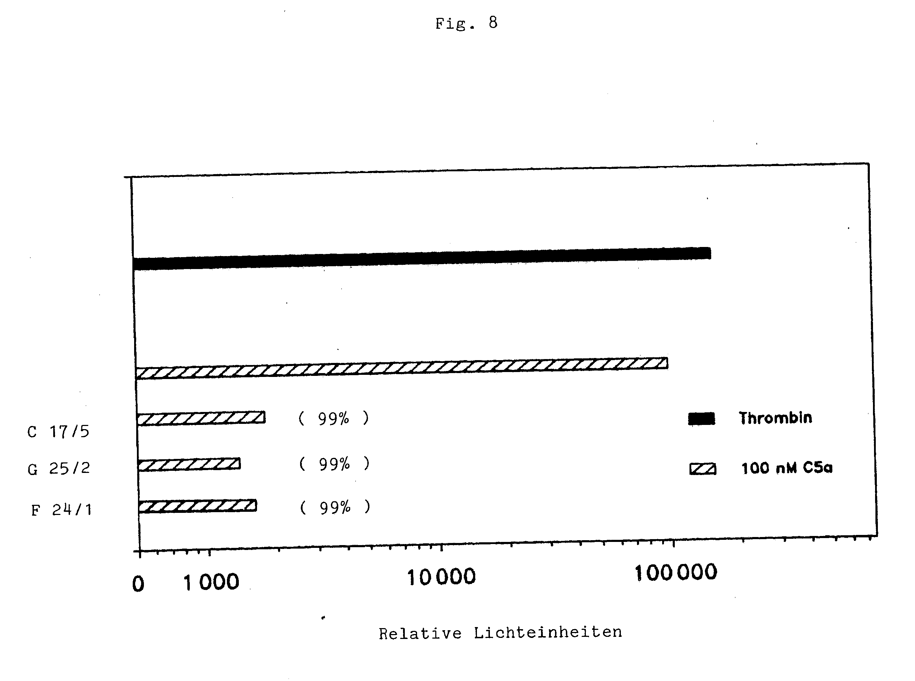

Fig. 8 zeigt die Freisetzung von ATP aus Meerschweinchenblutplättchen, wie sie durch Thrombin oder C5a induziert werden kann. Es ist allgemein bekannt, daß Thrombin die stärkste Freisetzungsreaktion auslöst (Messung der ATP-Freisetzung über ein Biolumineszenzverfahren). 100 nM C5a induziert eine fast ebenso starke ATP-Freisetzung wie Thrombin. Die Wirkung von C5a kann durch vorherige Inkubation mit den mAK C17/5 oder G25/2 auf etwa 1% reduziert werden.

Die Tatsache, daß beide Antikörper, die in dem neuen ELISA verwendet werden, C17/5 und G25/2 die biologische Funktion von C5a, d.h. die Rezeptorbindung inhibieren (wie in separaten Versuchen mit Hilfe von Fluoreszenz-optischen Verfahren nachgewiesen werden konnte) erscheint zunächst verwunderlich, da die Bindung der beiden Antikörper an C5a unabhängig voneinander erfolgt, sie sich also nicht gegenseitig blockieren. Eine genaue Erklärung kann dazu z.Zt. nicht gegeben werden. Jedoch wird angenommen, daß C5a an den zellulären Rezeptor mit mehreren Bereichen auf dem Molekül bindet (Arbeiten von Hugli et al., 1984, und Mollison et al., 1989). Es erscheint also möglich, daß, sollte sich diese These bewahrheiten, die beiden Antikörper an verschiedenen Bereichen des C5a Moleküls, die beide für die Rezeptorbindung notwendig sind, ansetzen. Andererseits könnte es sich um eine einzige größere Rezeptorbindungsstelle auf dem C5a Molekül handeln, die einen als Neoepitop exprimierten Teil hat und einen anderen, der auch auf dem Muttermolekül (C5) vorhanden ist. Beide Bereiche dieser Rezeptorbindungsstelle wären für die Assoziation für C5a mit seinem Rezeptor notwendig. Die Blockade nur eines Bereiches reichte aus, um die Bindung an den Rezeptor zu verhindern.

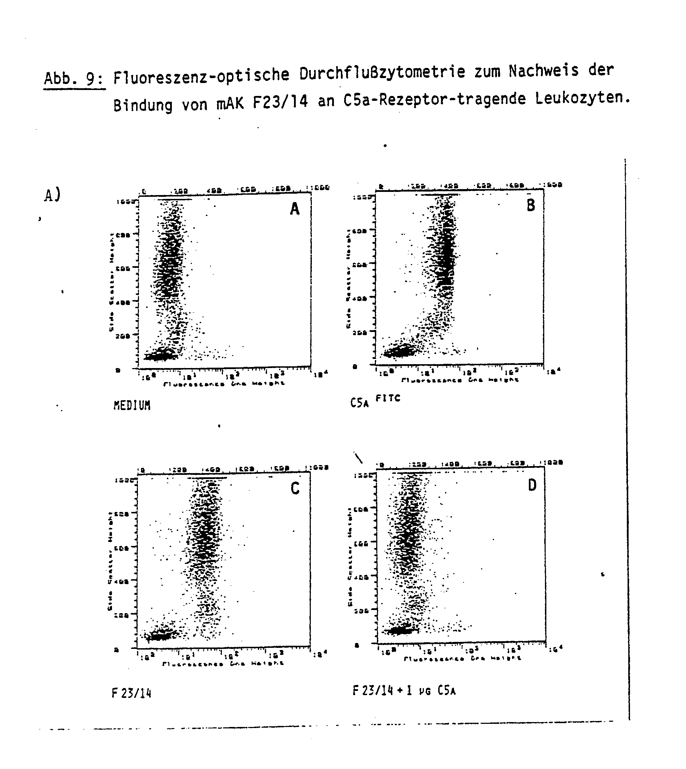

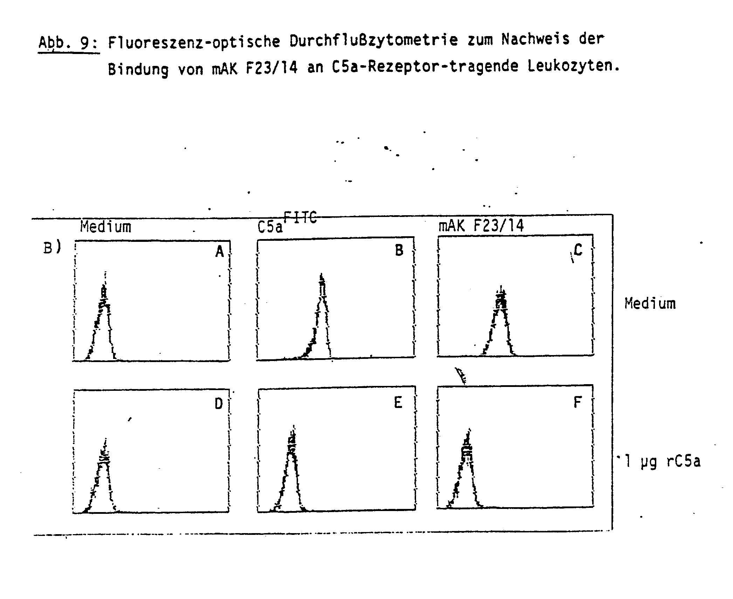

Fig. 9A zeigt eine Fluoreszenz-optische Durchflußcytometrie zum Nachweis der Bindung von mAK F23/14 an C5a-Rezeptor-tragende Leukozyten. Der dieser Figur zugrundeliegende Versuch ist in Beispiel 3 beschrieben. Im einzelnen zeigen:

Rahmen A - unbehandelte Zellen

Rahmen B - Blutzellen nach Behandlung mit Fluorescein-markiertem C5a

Rahmen C - Blutzellen nach Behandlung mit dem anti-idiotypischen Antikörper F23/14 und dem Nachweis dieses Antikörpers mittels eines Fluorescein-markierten Anti-Maus-Immunglobulins

Rahmen D - Blutzellen nach einer Behandlung wie in C beschrieben, der jedoch eine Vorbehandlung mit rekombinantem C5a vorgeschaltet wurde.

Fig. 9B zeigt den in Fig. 9A dargestellten Versuch bei Auswertung ausschließlich der Granulozyten.

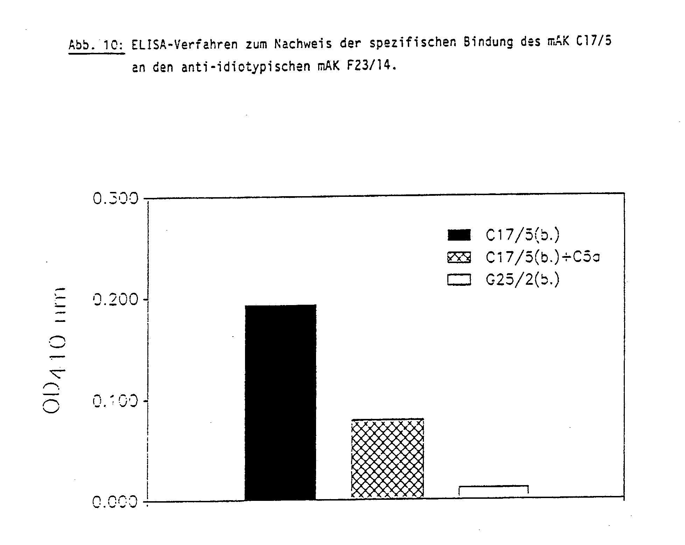

Fig. 10 zeigt die Ergebnisse eines ELISA-Verfahrens zum Nachweis der spezifischen Bindung des mAK C17/5 an den anti-idiotypischen mAK F23/14.

Jeweils 1 ug mAK F23/14 wurde in den Näpfen einer Mikrotiterplatte adsorbiert (in 50 mM Na-Carbonat, pH 10,6; 16 Stunden bei 22° C). Freie Bindungsstellen der Plastikoberfläche wurden durch Inkubation mit je 0,2 ml PBS-Gelatine (1% Gew./Vol.) für 30 Minuten bei 22° C blockiert. Anschließend wurde einmal mit 0,250 ml PBS-Tween 20 (0,05% Gew./Vol.) gewaschen und mit jeweils 0,1 ml (= 10 ug) mAK C17/5-Biotin oder mAK G25/2-Biotin oder mAK C17/5-Biotin nach einer Vorinkubation mit dem 10fachen molaren Überschuß an C5a (1 Stunde auf Eis) für 2 Stunden bei 22° C inkubiert. Nach zweimaligem Waschen mit PBS-Tween 20 (wie oben) wurde 0,1 ml 1/500 verdünntes Streptavidin-Peroxidase Konjugat (Fa. Amersham, Braunschweig) hinzugegeben und 1 Stunde bei 22° C inkubiert. Darauf folgten 4 Waschschritte mit PBS-Tween 20 (wie oben) und die Zugabe des Peroxidase-Substrates ABTS. Die nach 10 Minuten bei 22° C entwickelte Farbintensität wurde im Photometer bei 410 nm (gegen die Referenzwellenlänge 490 nm) gemessen.

Die rechte Säule (schwarz) stellt die Bindung von C17/5 an den in die Platte adsorbierten mAK F23/14 dar. Nach Vorinkubation von C17/5 mit dem Antigen C5a hat C17/5 die Fähigkeit, an F23/14 zu binden, bei einem nur 10fachen Überschuß von C5a zu 60% verloren (schraffierte Säule). Der mAK G25/2, der ebenfalls an C5a bindet und die Rezeptorbindung von C5a dadurch verhindert, wird von F23/14 nicht erkannt (offene Säule).

Beispiel 1:

Herstellung des monoklonalen Antikörpers C17/5

1.1 Vorbereitung des antigenen Materials für die Immunisierung von Mäusen

Bei der Vorbereitung des antigenen Materials für die Immunisierung von Mäusen wurde Wert darauf gelegt, daß das zur Immunisierung eingesetzte C5a dem Immunsystem mit freier Rezeptor-Bindungsstelle präsentiert werden würde. Dazu wurde wie folgt vorgegangen:

Ein monoklonaler Antikörper, der nach Bindung an C5a dessen biologische Wirkung nicht oder kaum inhibiert, wurde selektioniert. Solche Antikörper sind nach üblichen Methoden durch Immunisierung mit C5 oder C5a/C5adesarg, gegebenenfalls auch mit synthetischen Peptiden, leicht erhältlich. 5 mg isolierten monoklonalen Antikörpers M4/1 (eigene Produktion) wurden an jeweils 1 ml aktiviertes Sepharosegel gekoppelt. Die Aktivierung des Sepharosegels und die Kupplung erfolgte exakt nach den Angaben des Herstellers (Pharmacia). Die Antikörper tragenden Gelpartikel wurden anschließend mit 5 ug C5a/ml phosphatgepufferter Kochsalzlösung (PBS), enthaltend 135 mM NaCl, 20 mM Na-Phosphat, pH 7,2, 30 Minuten lang inkubiert, um den an das Gel fixierten Antikörper mit C5a zu sättigen. Die beladenen Sepharosepartikel wurden anschließend mit PBS - 0,1% (Gew./Vol.) Gelatine gewaschen, um freies C5a herauszuwaschen.

Die Gelpartikel mit dem gekoppelten Anti-C5a-Antikörper, der mit C5a gesättigt worden war, wurden jetzt mit Zellen der Linie U 937 (Barker et al., 1986, und Chenoweth et al., 1984) inkubiert. Die Zellen waren vorher in Gegenwart von 500 uM Dibutyryl-cAMP vier Tage kultiviert worden, um die Anzahl der auf diesen Zellen exprimierten C5a-Rezeptoren zu erhöhen (Barker et al., 1986, und Chenoweth et al., 1984). Ca. $5 \times 10^6$ Zellen in 1 ml Medium (72% RPMI 1640, 18% Medium 199, 10% fötales Kälberserum, $5 \times 10^{-5}$ M Colchicin) wurden mit 20 ul gepackter Sepharosepartikel 1 Stunde bei 37°C auf einer Rotationsapparatur vorsichtig gemischt. Die mikroskopische Kontrolle zeigte, daß die Gelpartikel adhärent an die Zelloberflächen gebunden hatten, das heißt, daß das von dem Antikörper präsentierte C5a mit dem zellulären C5a-Rezeptor wechselwirkte.

Das Gemisch aus Zellen und Gelpartikeln wurde anschließend durch 5 Minuten Zentrifugation bei 800 g pelletiert, der Überstand wurde abgenommen und das Pellet in 1 ml Lysepuffer gegeben (Zusammensetzung: 120 mM NaCl, 50 mM Tris-HCl, pH 8,0, 5 mM EDTA, 1 mM APMSF, 1000 KI Trasylol, 0,1 mM Pepstatin, 0,1 mM Leustatin, 1 mg Chymostatin per 100 ml, 1% (Gew./Vol.) NP 40, 0,05% (Gew./Vol.) Triton X-100 und $5 \times 10^{-5}$ M Colchicin). Die Suspension wurde 2 Stunden bei 4°C End über End rotiert. Im Anschluß daran wurde die gesamte Mischung auf ein Lymphoprepdichtemedium (Dichte 1,077 g/cm³) gegeben und 5 Minuten bei 4°C und 500 UpM zentrifugiert. Nach der Zentrifugation befanden sich die Zellen und Zellreste an der Interphase zwischen dem Gradientenmaterial und dem überschichteten Medium, während die Sepharosepartikel am Röhrchenboden pelletiert waren. Sepharosepartikel und Zellreste wurden getrennt entnommen.

1.2 Immunisierung von Mäusen

Die Sepharosepartikel wurden dreimal in Lysepuffer (s.o.) gewaschen, wobei die NP-40 Konzentration bei jedem Waschschritt um 50% erniedrigt wurde. Die letzte Waschung erfolgte in Abwesenheit von NP-40. Die so behandelten Sepharosepartikel wurden nun als antigenes Material für die Immunisierung von BALB/c Mäusen und die Herstellung monoklonaler Antikörper aus den Milzzellen der immunisierten Tiere verwendet. Die Mäuse wurden mit Sepharose-C5a Partikeln, die $5 \times 10^7$ U 937 Zellen entsprachen, immunisiert, d.h. mit 200 ul gepackten, Membranrezeptor-beladenen Sepharosepartikeln. Diese Menge der Sepharose wurde in 0,5 ml PBS in Mäuse intraperitoneal injiziert. Die Injektionen wurden 4 Wochen und 8 Wochen nach der ersten Injektion wiederholt. Drei Wochen später wurden die Milzzellen entnommen und mit Myelomzellen fusioniert. Fünf Tage vor der Gewinnung der Milzzellen zur Fusion wurde die Immunisierung mit jeweils derselben Dosis an vier aufeinanderfolgenden Tagen fortgeführt. Am fünften Tag wurden die Milzzellen entnommen und die Fusion nach dem dem Fachmann bekannten Verfahren mit X63-Ag.8.653 Zellen (Köhler und Milstein, 1975 und Peters, Baumgarten und Schulze, Monoklonale Antikörper, Springer-Verlag, 1985) durchgeführt. Die gesamte Immunisierungszeit betrug damit 11 Wochen.

Eine Vielzahl verschiedener monoklonaler Antikörper gegen C5a und möglicherweise auch gegen den C5a-Rezeptor, der in solubilisierter Form an die C5a tragenden Sepharosepartikel gebunden hatte, konnte identifiziert und kloniert werden. Einer dieser Antikörper war der Antikörper C17/5, der, wie später analysiert, natives C5 nicht bindet, jedoch sowohl C5a als auch C5adesarg bindet. Der Antikörper ist also spezifisch für ein Neoepitop auf C5a/C5adesarg, das im nativen Molekül nicht zugänglich ist. Die Identifizierung des erkannten Neoepitopes als Bindungsstelle des C5a-Rezeptors ist in Beispiel 3 gezeigt.

Beispiel 2

Bestimmung des C5a-Gehaltes in EDTA-Plasma oder aktiviertem Humanserum

2.1 Bildung der Matrix-Antikörper-Antigen-Antikörper-Komplexe

Für die Durchführung des erfindungsgemäßen Verfahrens werden zunächst jeweils 100 ul einer Lösung des monoklonalen Antikörpers C17/5 (40 ug/ml) in die Näpfe einer Mikrotiterplatte geschichtet. Die Platte wird 16 Stunden bei 22° C inkubiert, anschließend wird die antikörperhaltige Lösung abgesaugt. Zu den Näpfen werden je 200 ul von PBS, enthaltend 1% (Gew./Vol.) Gelatine, zugegeben und 20 Minuten bei Raumtemperatur (22° C) inkubiert. Danach wird die PBS-Gelatine-Lösung abgesaugt und einmal mit 250 ul PBS, enthaltend 0,05% Tween-20, gewaschen. In die so blockierten Näpfe der Mikrotiterplatte wird jetzt die jeweilige Probe (beispielsweise 1:2 oder stärker verdünntes EDTA-Plasma, aktiviertes Humanserum in höheren Verdünnungen, andere Körperflüssigkeiten) eingefüllt (jeweils 100 ul Probe) und nach einer Inkubationszeit von 2 Stunden bei 22° C, während derer das C5a an den matrixgebundenen Antikörper binden soll, abgesaugt. Überschüssiges Material wird durch zweifaches Waschen mit PBS-Tween 20 (wie oben) ausgewaschen. Das nunmehr an den Antikörper C17/5 gebundene C5a wird in einem weiteren Inkubationsschritt mit jeweils 100 ul G25/2 (1 ug/ml PBS-Tween-20) inkubiert, wobei dieser Antikörper zuvor biotinyliert worden ist (Schulze und Götze, 1986). Nach einstündiger Inkubation bei 22° C wird auch die zweite Antikörperlösung abgesaugt und die nun gebildeten Matrix-fixierten Antikörper-Antigen-Antikörperkomplexe dreimal mit je 250 ul PBS-Tween-20 gewaschen.

2.2 Detektion des gebundenen zweiten Antikörpers G25/2

Die im Beispiel 2.1 gebildeten Matrix-fixierten Antikörper-Antigen-Antikörper-Komplexe werden mit Hilfe eines Streptavidin-Peroxidase-Konjugats und der Farbreaktion, die mit Hilfe eines Peroxidase-Substrates erzielt werden kann, nachgewiesen. Dabei wird wie folgt vorgegangen:

100 ul des 1/1000 mit PBS-Tween-20 (0,05% Vol/Vol) verdünnten Streptavidin-Peroxidase-Konjugates der Fa. Amersham, Braunschweig, werden in jeden Napf eingefüllt. Es folgt eine Inkubation von einer Stunde bei 22° C. Danach wird 4 x mit je 250 ul PBS-Tween-20 (wie oben) gewaschen. Anschließend werden 100 ul einer Lösung zugesetzt, die die folgende Zusammensetzung hat: 0,02 mM ABTS, 100 mM Natriumacetat, 50 mM Natriumphosphat, 2,5 mM $H_2O_2$, pH 4,2. Die optische Dichte der sich in den Näpfen entwickelnden Farblösung wird in einem Plattenphotometer bei 410 nm gegen die Referenzwellenlänge von 490 nm alle drei Minuten gemessen und der Anstieg der optischen Dichte (OD) pro Minute im linearen Bereich des Anstieges berechnet. Auf jeder Mikrotiterplatte wird ein kalibrierter C5a Standard mitgeführt und eine Eichkurve hergestellt. Von dieser Eichkurve läßt sich dann der Gehalt der untersuchten biologischen Flüssigkeit an C5a aufgrund der erzielten optischen Dichte pro Minute ablesen (s. Fig. 2).

2.3 Anwendung des Verfahrens bei 50 gesunden Probanden

Mit dem erfindungsgemäßen Verfahren konnte erstmals die Normalkonzentration von C5a im EDTA-Plasma gesunder Probanden bestimmt werden. Dafür wurden 50 Probanden je 10 ml Blut entnommen. Das Blut wurde in Plastikröhrchen, in denen sich trockenes Kalium-EDTA befand (Fa. Sarstedt, Nümbrecht) aufgefangen, so daß die Endkonzentration des EDTA 4 mM betrug. Das Blut wurde sofort nach der Gewinnung 20 Minuten lang bei 1000 g zentrifugiert und das Plasma mit einer Pasteurpipette abgehebert. Das Plasma wurde sofort auf Eis verbracht und dann in Aliquots bei -70° C eingefroren. Vor den Analysen auf den Gehalt von C5a wurde ein Aliquot des eingefrorenen Plasmas bei 37° C aufgetaut und dann sofort in ein Eisbad verbracht. Aus diesen eisgekühlten Aliquots wurden Proben für die Bestimmung der C5a-Konzentrationen entnommen. Bei den normalen Blutspendern bzw. dem gesunden Laborpersonal wurde das EDTA-Plasma 1:2 verdünnt (Verdünnungsflüssigkeit: PBS-Tween-20 wie oben, aber enthaltend 20 mM EDTA) eingesetzt. Die Ergebnisse sind in Fig. 4 dargestellt. Es ergab sich ein mittlerer Wert von 1,026 ± 0,385 mg C5a/ml für unverdünntes EDTA-Plasma gesunder Probanden.

Beispiel 3:

Nachweis der Bindung von mAK F23/14 an C5a-Rezeptor-tragende Leukozyten

Mit PBS gewaschene Blutzellen aus 2 ml Blut wurden mit Gey'scher Lösung behandelt, um die Erythrozyten zu lysieren. Dabei wurde genau nach Mishell und Saiigi, 1980, vorgegangen.

Die Leukozyten wurden durch einmaliges Waschen in der Zentrifuge mit PBS (5 Minuten bei 500 g) von den Erythrozytenresten befreit. Sie wurden dann zur Blockade der IgG-Fc Rezeptoren in Kaninchen IgG-Lösung aufgenommen und 20 Minuten bei 4°C inkubiert. Die Kaninchen IgG-Lösung bestand aus: 2 mg IgG/ml PBS-Gelatine (1% Gew./Vol.), 20 mM Na-Azid. Danach wurde einmal mit PBS-Gelatine-Azid gewaschen (wie oben) und wieder in PBS-Gelatine-Azid Kaninchen IgG aufgenommen. Die Zellsuspension wurde auf 6 Plastikröhrchen verteilt. Je 3 Röhrchen wurden mit 0,1 ml Medium (RPMI 72%; Medium 199 18%; FKS 10%) oder mit 0,1 ml rekombinantem C5a (1 ug) 1 Stunde auf Eis inkubiert. Danach wurde wieder gewaschen und mit Medium C5a$^{FITC}$ oder mAK F23/14 inkubiert (1 Stunde, Eisbad).

Dann wurden die Zellen in jedem Röhrchen zweimal mit PBS-Gelatine-Azid gewaschen und der Fluoreszenz-optischen Analyse (FacStar plus, Fa. Becton Dickinson) zugeführt. Röhrchen mit mAK F23/14 wurden vorher mit 0,5 ug Anti-Maus IgG/IgM der Fa. Paesel, Frankfurt, inkubiert (1 Stunde auf Eis; danach 4mal waschen mit PBS-Gelatine-Azid). Diese Antikörperpräparation lag als IgG F(ab')2 Fragmente vor, die FITC-konjugiert waren.

Die Ergebnisse der fluoreszenz-optischen Durchflußcytometrie sind in Fig. 9A und 9B gezeigt. Im einzelnen zeigen:

Fig. 9A, Rahmen A:

Unbehandelte Zellen haben eine Basisfluoreszenz von maximal etwa $0,5 \times 10^1$ relativen Einheiten. Es sind drei verschiedene Populationen zu unterscheiden. Granulozyten mit einem sehr starken Side Scatter und niedriger Fluoreszenz, Monozyten mit geringerhöhter Fluoreszenz und einem verstärkten Side Scatter und Lymphozyten mit niedriger Basisfluoreszenz und sehr niedrigem Side Scatter.

Fig. 9A, Rahmen B:

Nach Behandlung der Blutzellen mit Fluorescein-markiertem C5a zeigen Granulozyten und Monozyten ein verstärktes Fluoreszenzsignal von über $10^1$ relativen Einheiten. Die Position der Lymphozyten hat sich nicht verändert. Dies bedeutet, daß das C5a nur an Monozyten und Granulozyten gebunden hat.

Fig. 9A, Rahmen C:

Die Behandlung der Leukozyten mit dem anti-idiotypischen (anti-C17/5) Antikörper und der Nachweis dieses Antikörpers mit Hilfe eines Fluorescein-markierten Anti-Maus-Immunglobulins zeigt ein fast identisches Bild wie das, das durch C5a$^{FITC}$ erzielt wurde. Das heißt, daß der Antikörper F23/14 an dieselben Zellpopulationen in ähnlicher Weise bindet wie der Ligand C5a selbst.

Fig. 9A, Rahmen D:

Die Bindung des anti-idiotypischen mAK F23/14 kann durch Vorbehandlung der Blutleukozyten mit einem Mikrogramm rekombinantem C5a blockiert werden. Nach dieser Vorbehandlung bindet der mAK F23/14 nicht mehr, der Rahmen D ist von dem Rahmen A (Mediumkontrolle) nicht unterscheidbar.

Fig. 9B:

Hier sind die Histogramme der oben beschriebenen Analyse noch einmal in anderer Form dargestellt. Die Hintergrundfluoreszenz von Zellen, die mit nicht markiertem rekombinantem C5a behandelt worden sind, unterscheidet sich nicht von der Fluoreszenz, die durch Mediumbehandlung allein erreicht wird (Rahmen A und D). Die Behandlung der Zellen mit C5a$^{FITC}$ führt zu einer erhöhten Fluoreszenz (Verschiebung nach rechts, B), die durch Vorbehandlung mit rekombinantem C5a verhindert wird (E). Die Behandlung der Zellen mit dem anti-idiotypischen Antikörper F23/14 (C) ergibt ein ähnliches Bild wie die Behandlung mit C5a$^{FITC}$ (B). Die Vorbehandlung der Zellen mit 1 Mikrogramm rekombinantem C5a verhindert die Bindung des mAK F23/14 (F), d.h. es wird ein den Kontrollen (A, D und E) gleiches Bild erhalten.

Bei dieser Auswertung wurden nur die Granulozyten analysiert, d.h. es wurde das "Granulozytenfenster" des Analysengerätes zur Auswertung verwendet.

**Liste der zitierten Literatur**

1) Barker, Jose, Williams & Burton, Biochem. J. 236 , 621, 1986

2) Chenoweth, Soderberg & von Wedel, J. Leuk. Biol. 36 , Abstr. 241, 1984

3) Hugli, Springer Semin. Immunopathol. 7 , 193-219, 1984

4) Köhler & Milstein, Nature 256, 495-497, 1975

5) Mishell & Saiigi, Selected Methods in Cellular Immunology, S. 23-24, W.H. Freeman, San Francisco, 1980

6) Mollison, Mandecki et al., Proc. Natl. Acad. Sci. USA 86 , 292-296, 1989

7) Peters, Baumgarten & Schulze, Monoklonale Antikörper, Springer Verlag, 1985

8) Schulze & Götze, Complement 3 , 25-39, 1986

9) Stevens, O'Hanley, Shapiro, Mihm, Satoh, Collins & Raffin, J. Clin. Invest. 77 , 1812-1816, 1986

10) Takeda, Kinoshita, Takata, Kozono, Tanaka, Hong & Inoue, J. Immunological Methods 101 , 265-270, 1987

11) Vogt, Complement 3 , 177, 1986.

## Ansprüche

1. Verfahren zum Nachweis und/oder der quantitativen Bestimmung von Komplementpeptid C5a und/oder C5adesarg in biologischen Flüssigkeiten, **dadurch gekennzeichnet,** daß es die folgenden Schritte umfaßt:

a) In-Berührung-bringen einer Probe der biologischen Flüssigkeit, die C5a und/oder C5adesarg enthält, mit einer Matrix, auf der eine erste Antikörper-Präparation (Akp 1), enthaltend Antikörper, die C5a und/oder C5adesarg binden, fixiert ist,

b) In-Berührung-bringen der aus a) resultierenden beladenen Matrix mit einer zweiten Antikörper-Präparation (Akp 2), enthaltend Antikörper und/oder Teile davon, die an natives Komplementprotein C5 und/oder an C5a und/oder C5adesarg binden, wobei die gebundenen Antikörper und/oder Antikörperteile aus der zweiten Antikörper- Präparation detektierbar sind,

c) Detektieren der gebundenen Antikörper und/oder Antikörperteile aus der zweiten Antikörper-Präparation.

2. Verfahren nach Anspruch 1, **dadurch gekennzeichnet,** daß die erste und/oder zweite Antikörper-Präparation (Akp 1 und/oder Akp 2) monoklonale Antikörper bzw. Antikörperteile bevorzugt aus Ratten, Kaninchen, Schweinen oder Affen, besonders bevorzugt aus Mäusen oder Menschen umfaßt.

3. Verfahren nach Anspruch 2, **dadurch gekennzeichnet,** daß die monoklonalen Antikörper bzw. Antikörperteile der ersten oder zweiten Antikörperpräparation im Komplementpeptid C5a und/oder C5adesarg an die Rezeptorbindungsstelle für den C5a-spezifischen Rezeptor, jedoch nicht an die korrespondierende Aminosäuresequenz im nativen C5-Molekül binden.

4. Verfahren nach Anspruch 2 oder 3, **dadurch gekennzeichnet,** daß die erste Antikörper-Präparation (Akp 1) den monoklonalen Antikörper C17/5 (CNCM I-887) und/oder funktionell äquivalente Antikörper umfaßt.

5. Verfahren nach mindestens einem der Ansprüche 1 bis 4, **dadurch gekennzeichnet,** daß die zweite Antikörper-Präparation (Akp 2) den monoklonalen Antikörper G25/2 (CNCM I-889) und/oder Teile davon und/oder funktionell äquivalente Antikörper umfaßt.

6. Verfahren nach mindestens einem der Ansprüche 1 bis 5, **dadurch gekennzeichnet,** daß die Antikörper und/oder Antikörperteile der zweiten Antikörper-Präparation direkt detektierbar sind, vorzugsweise durch Fluoreszenz, Radioaktivität oder Chemilumineszenz.

7. Verfahren nach mindestens einem der Ansprüche 1 bis 5, **dadurch gekennzeichnet,** daß die Antikörper und/oder Antikörperteile der zweiten Antikörper-Präparation indirekt detektierbar sind, vorzugsweise durch die enzymatische Aktivität mindestens eines Markerenzymes.

8. Verfahren nach Anspruch 7, **dadurch gekennzeichnet,** daß das Markerenzym alkalische Phosphatase, $\beta$-Galaktosidase, Mikroperoxidase oder Meerrettichperoxidase ist.

9. Verfahren nach Anspruch 7 und/oder 8, **dadurch gekennzeichnet,** daß die Antikörper und/oder Antikörperteile der zweiten Antikörper-Präparation biotinyliert sind und durch In-Berührung-bringen mit modifiziertem Avidin oder Streptavidin nachgewiesen werden.

10. Verfahren nach Anspruch 7, **dadurch gekennzeichnet,** daß die Antikörper und/oder Antikörperteile der zweiten Antikörperpräparation aus einer anderen Säugetierspecies als die der ersten Antikörperpräparation stammen und durch Reaktion mit einer dritten, detektierbaren Antikörperpräparation nachgewiesen werden, deren Antikörper spezifisch mit den Antikörpern bzw. Antikörperteilen der zweiten Antikörperpräparation reagieren.

11. Verfahren nach mindestens einem der Ansprüche 1 bis 10, **dadurch gekennzeichnet,**

daß es eine Nachweisempfindlichkeit von 20 pg/ml hat.

12. Testkit für die Durchführung eines Verfahrens zum Nachweis und/oder der quantitativen Bestimmung von C5a und/oder C5adesarg in biologischen Flüssigkeiten, umfassend

a) eine erste Antikörper-Präparation (Akp 1), enthaltend Antikörper, die an C5a und/oder C5adesarg binden, wobei die erste Antikörper-Präparation gegebenenfalls bereits an eine Matrix gekoppelt vorliegt,

b) eine zweite Antikörper-Präparation (Akp 2), enthaltend Antikörper und/oder Teile davon, die an natives Komplementprotein C5 und/oder C5a und/oder C5adesarg binden, wobei die gebundenen Antikörper und/oder Antikörperteile aus der zweiten Antikörper-Präparation detektierbar sind,

c) gegebenenfalls ein oder mehrere Nachweisreagenzien, enthaltend Enzyme und/oder Substrate und/oder eine dritte Antikörperpräparation für die Detektionsreaktion.

13. Testkit nach Anspruch 12,
**dadurch gekennzeichnet,**
daß die erste und/oder zweite Antikörper-Präparation monoklonale Antikörper bzw. Antikörperteile bevorzugt aus Ratten, Kaninchen, Schweinen oder Affen, besonders bevorzugt aus Mäusen oder Menschen umfaßt.

14. Testkit nach Anspruch 12 oder 13,
**dadurch gekennzeichnet,**
daß die monoklonalen Antikörper bzw. Antikörperteile der ersten oder zweiten Antikörperpräparation im Komplementpeptid C5a und/oder C5adesarg an die Rezeptorbindungsstelle für den C5a-spezifischen Rezeptor, jedoch nicht an die korrespondierende Aminosäuresequenz im nativen C5-Molekül binden.

15. Testkit nach mindestens einem der Ansprüche 12 bis 14,
**dadurch gekennzeichnet,**
daß die erste Antikörper-Präparation (Akp 1) den monoklonalen Antikörper C17/5 (CNCM I-887) und/oder funktionell äquivalente Antikörper umfaßt.

16. Testkit nach mindestens einem der Ansprüche 12 bis 15,
**dadurch gekennzeichnet,**
daß die zweite Antikörper-Präparation (Akp 2) den monoklonalen Antikörper G25/2 (CNCM I-889) und/oder Teile davon und/oder funktionell äquivalente Antikörper umfaßt.

17. Testkit nach mindestens einem der Ansprüche 12 bis 16,
**dadurch gekennzeichnet,**
daß die Antikörper und/oder Antikörperteile der zweiten Antikörper-Präparation direkt, vorzugsweise durch Fluoreszenz, Radioaktivität oder Chemilumineszenz detektierbar sind.

18. Testkit nach mindestens einem der Ansprüche 12 bis 17,
**dadurch gekennzeichnet,**
daß die Antikörper und/oder Antikörperteile der zweiten Antikörper-Präparation indirekt, vorzugsweise durch die enzymatische Aktivität mindestens eines Markerenzymes, detektierbar sind.

19. Testkit nach Anspruch 18,
**dadurch gekennzeichnet,**
daß das Markerenzym alkalische Phosphatase, β-Galaktosidase, Mikroperoxidase oder Meerrettichperoxidase ist.

20. Testkit nach mindestens einem der Ansprüche 12 bis 19,
**dadurch gekennzeichnet,**
daß die Antikörper und/oder Antikörperteile der zweiten Antikörper-Präparation biotinyliert sind und durch In-Berührung-bringen mit modifiziertem Avidin nachgewiesen werden.

21. Testkit nach Anspruch 18,
**dadurch gekennzeichnet,**
daß die Antikörper und/oder Antikörperteile der zweiten Antikörperpräparation aus einer anderen Säugetierspecies als die der ersten Antikörperpräparation stammen und durch Reaktion mit einer dritten, detektierbaren Antikörperpräparation nachgewiesen werden, deren Antikörper spezifisch mit den Antikörpern bzw. Antikörperteilen der zweiten Antikörperpräparation reagieren.

22. Zellinie,
**dadurch gekennzeichnet,**
daß sie einen monoklonalen Antikörper sezerniert, der im Komplementpeptid C5a und/oder C5adesarg an die Rezeptorbindungsstelle für den C5a-spezifischen Rezeptor, jedoch nicht an die korrespondierende Aminosäuresequenz im nativen C5-Molekül bindet.

23. Zellinie nach Anspruch 22,
**dadurch gekennzeichnet,**
daß sie den monoklonalen Antikörper C17/5 sezerniert und bei der CNCM mit der Hinterlegungsnummmer I-887 hinterlegt ist.

24. Zellinie,
**dadurch gekennzeichnet,**
daß sie den monoklonalen Antikörper G25/2 sezerniert und bei der CNCM mit der Hinterlegungsnummer I-889 hinterlegt ist.

25. Monoklonaler Antikörper mit spezifischer Bindung an C5a und/oder C5adesarg,
**dadurch gekennzeichnet,**
daß er im Komplementpeptid C5a und/oder C5adesarg an die Rezeptorbindungsstelle für den C5a-spezifischen Rezeptor, jedoch nicht an die korrespondierende Aminosäuresequenz im nativen C5-Molekül bindet.

26. Monoklonaler Antikörper nach Anspruch 25,
**dadurch gekennzeichnet,**
daß seine variable Region und/oder seine konstante Region aus einem Säugetier stammt.

27. Monoklonaler Antikörper nach Anspruch 26,
**dadurch gekennzeichnet,**
daß das Säugetier Ratte, Kaninchen, Schwein oder Affe, bevorzugt Maus oder Mensch ist.

28. Monoklonaler Antikörper nach Anspruch 27,
**dadurch gekennzeichnet,**
daß seine konstante Region humanen Ursprungs ist.

29. Monoklonaler Antikörper nach mindestens einem der Ansprüche 25 bis 28,
**dadurch gekennzeichnet,**
daß er vom IgG- oder IgM-Typ ist.

30. Monoklonaler Antikörper nach Anspruch 25,
**dadurch gekennzeichnet,**
daß er von der Zellinie nach Anspruch 23 sezerniert wird.

31. Monoklonaler Antikörper,
**dadurch gekennzeichnet,**
daß er an C5, C5a und/oder C5adesarg bindet und von der Zellinie nach Anspruch 24 sezerniert wird.

32. Zusammensetzung,
**dadurch gekennzeichnet,**
daß sie mindestens einen monoklonalen Antikörper nach einem der Ansprüche 25 bis 30 und/oder Anspruch 31 enthält.

33. Verwendung einer Zusammensetzung nach Anspruch 32 zur Therapie und Prophylaxe von Erkrankungen, die auf einem erhöhten C5a-Spiegel im Blut des Patienten beruhen, bevorzugt bei ARDS, Sepsis, Schock und anderen Zuständen bei intra- und extravasculärer Komplementaktivierung.

34. Verfahren zum Herstellen von Hybridoma-Zellinien, die ein nach proteolytischer Spaltung von C5 auf C5a exponiertes Neoepitop erkennen, umfassend die folgenden Schritte:

a) Inkubieren von C5a mit einem gegebenenfalls an einen Träger gekoppelten Antikörper, dessen Bindung an C5a C5a-spezifische Aktivitäten nicht oder nur geringfügig beeinflußt,

b) gegebenenfalls Inkubieren des an den Antikörper gebundenen C5a's mit Zellen, die C5a-Rezeptoren aufweisen, Anreichern der Antikörper-C5a-Zellaggregate, Lysieren der Zellen, Wiedergewinnen der Antikörper-C5a-Komplexe, bevorzugt durch Dichtegradienten-Zentrifugation,

c) Immunisieren eines Säugetieres mit den gegebenenfalls angereicherten Antikörper-C5a-Komplexen,

d) Gewinnen von Antikörper-produzierenden Zellen aus dem immunisierten Säugetier, bevorzugt von Milzzellen, und Immortalisieren dieser Zellen in an sich bekannter Weise.

35. Monoklonaler Antikörper,
**dadurch gekennzeichnet,**
daß er ein anti-idiotypischer Antikörper gegen einen Antikörper nach einem der Ansprüche 25 bis 30 ist.

36. Monoklonaler Antikörper nach Anspruch 35,
**dadurch gekennzeichnet,**
daß seine variable Region und/oder seine konstante Region aus einem Säugetier stammt.

37. Monoklonaler Antikörper nach Anspruch 36,
**dadurch gekennzeichnet,**
daß das Säugetier Ratte, Kaninchen, Schwein oder Affe, bevorzugt Maus oder Mensch ist.

38. Monoklonaler Antikörper nach Anspruch 27,
**dadurch gekennzeichnet,**
daß seine konstante Region humanen Ursprungs ist.

39. Monoklonaler Antikörper nach mindestens einem der Ansprüche 35 bis 38,
**dadurch gekennzeichnet,**
daß er vom IgG- oder IgM-Typ ist.

40. Monoklonaler Antikörper nach Anspruch 35,
**dadurch gekennzeichnet,**
daß er von der Zellinie F23/14 mit der Hinterlegungsnummer CNCM Nr. I-888 sezerniert wird.

41. Verwendung eines monoklonalen Antikörpers nach mindestens einem der Ansprüche 35 bis 40 zur Blockierung des C5a-spezifischen Rezeptors.

42. Zellinie,
**dadurch gekennzeichnet,**
daß sie den monoklonalen Antikörper F23/14 sezerniert und bei der CNCM mit der Hinterlegungsnummer I-888 hinterlegt ist.

43. Verfahren zum Herstellen einer Zellinie mit den Eigenschaften der Zellinie nach Anspruch 42,
**dadurch gekennzeichnet,**
daß zur Immunisierung eines Säugetieres Hybridomzellen verwendet werden, die einen Antikörper mit den Eigenschaften der Antikörper nach Anspruch 25 bis 30 sezernieren.

44. Verfahren nach Anspruch 43,
**dadurch gekennzeichnet,**
daß die zur Immunisierung verwendeten Hybridomzellen vor der Verwendung zur Immunisierung bestrahlt werden, bevorzugt mit 1000 bis 5000 rad, besonders bevorzugt mit 2000 rad.

Fig. 1

EP 0 411 306 A1

Fig. 2
Eichkurve für die Quantifizierung von C5a

EP 0 411 306 A1

Serumverdünnung

C5a [ng/ml]

C5a-Bestimmung
in konzentrierten Plasma-/Serumproben

FIG. 3

EP 0 411 306 A1

Fig. 4

C5a—Normalwerte (n=50)

1,026± 0,385 ng C5a/ml

Fig. 5

$y=0,96x+0,10$
$r=0,98$

Methode B  C5a  [ng/ml]

Methode A  C5a  [ng/ml]

Serum−C5a [%]

C5a−Bestimmung in Plasmaproben
mit niedrigem Proteingehalt

Fig. 6

○ konv.
● neo

Serumverduennung [%]

FIG. 7

C5a [ng/ml]

C3r/C5a-Generation unter der in-vitro Aktivierung von Humanserum

Dauer der Aktivierung (Stunden)

C3a [ug/ml]

● C3a
○ C5a

Fig. 8

C 17/5 ( 99% )

G 25/2 ( 99% )

F 24/1 ( 99% )

Thrombin

100 nM C5a

0     1000          10000          100000

Relative Lichteinheiten

Abb. 9: Fluoreszenz-optische Durchflußzytometrie zum Nachweis der
Bindung von mAK F23/14 an C5a-Rezeptor-tragende Leukozyten.

<u>Abb. 9</u>: Fluoreszenz-optische Durchflußzytometrie zum Nachweis der Bindung von mAK F23/14 an C5a-Rezeptor-tragende Leukozyten.

Abb. 10: ELISA-Verfahren zum Nachweis der spezifischen Bindung des mAK C17/5 an den anti-idiotypischen mAK F23/14.

**Europäisches Patentamt**

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

EP 90 11 1920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 300 740 (CEFEM A/S) <br><br> * Ganzes Dokument * | 1-9,12-20,22-27,29-32,34 | G 01 N 33/68 <br> G 01 N 33/577 <br> C 12 N 5/12 <br> C 12 P 21/08 <br> A 61 K 39/395 |
| Y | -- | 35-44 | |
| Y | WO-A-88 07 058 (BIOSOLUTIONS PTY. LTD.) <br><br> * Ansprüche * <br><br> -- | 35-44 | |
| X | SCAND. J. IMMUNOL., Band 29, Nr. 3, März 1989, Seiten 333-341, Oxford, GB <br> K. BERGH et al.: "Measurement of complement activation in rabbit plasma or serum using monoclonal antibodies against C5a" <br><br> * Ganzes Dokument * <br><br> -- | 1-9,12-20,22-27,29-32 | RECHERCHIERTE SACHGEBIETE (Int. Cl.4) <br><br> G 01 N <br> C 12 P |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-32,34-44

Unvollständig recherchierte Patentansprüche:

Nicht recherchierte Patentansprüche: 33

Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 30-10-1990 | NOOIJ |

Europäisches
Patentamt **EUROPÄISCHER TEILRECHERCHENBERICHT**

| | EINSCHLÄGIGE DOKUMENTE | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| X | J. IMMUNOL. METHODS, Band 111, Nr. 2, 1988, Seiten 241-252, Elsevier, Amsterdam, NL<br>A. KLOS et al.: "Detection of native human complement components C3 and C5 and their primary activation peptides C3a and C5a anaphylatocis peptides by ELISA with monoclonal antibodies"<br><br>* Ganzes Dokument * | 1,2,4-9,11-13,15-20,24,31 | |
| D,X | J. IMMUNOL. METHODS, Band 101, Nr. 2, 1987, Seiten 265-270,Elsevier, Amsterdam, NL<br>J. TAKEDA et al.: "Rapid and simple measurement of human C5a-des-Arg level in plasma or serum using monoclonal antibodies"<br><br>* Ganzes Dokument * | 1-6,12,17,22,23,25-27,29,30,32 | |
| D,X | COMPLEMENT, Band 3, 1986, Seiten 25-39, S. Karger AG, Basel, CH, M. SCHULZE et al.: "A sensitive ELISA for the quantitation of human C5a in blood plasma using a monoclonal antibody"<br><br>* Ganzes Dokument * | 1,2,4-9,12,13,15-20,24,31 | |
| D,X | EP-A-0 245 993 (CETUS CORP.)<br>* Ansprüche * | 22,23,25-28,30,32 | |

RECHERCHIERTE SACHGEBIETE (Int. Cl. 4)